# EUROPEAN PATENT APPLICATION

(11) **EP 2 412 353 A2**
(43) Date of publication of application: **01.02.2012**
(21) Application number: 11174480.1
(22) Date of filing: 19.07.2011
(51) Int. Cl.: A61F 13/49

(54) **Panty with washable integrated absorbent element**

(30) Priority: 28.07.2010 IT MI20101388
(71) Applicant: ARTSANA S.p.A., 22070 Grandate (Como) (IT)
(72) Inventor: Montepara, Donatella Paola, 66036 ORSOGNA (Chieti) (IT); Leoni, Elena Stella, 22073 FINO MORNASCO (Como) (IT)
(74) Representative: Ripamonti, Enrico

(57) **Abstract**

The panty comprises a body (1) with a waist portion (2) to be positioned about the waist of the user wearing it, said portion having a front part (3), a rear part (4), and an intermediate portion (6) which extends between the front part (3) and rear part (4) by passing over the user's crotch, opposing openings (7) being present on its sides for the user's legs, an absorbent piece (10) being positioned to correspond with the user's intimate parts to absorb any fluid leakages from said parts; the absorbent piece (10) comprises a plurality of superimposed layer elements (13, 14, 16), the element for absorbing the fluid leakages being at least partly of wadding, the latter being a filamentous mass of fibres of average length between 5 and 55 mm, said body (1) being of natural material, the garment or panty being completely washable.

## Description

The present invention relates to a panty in accordance with the introduction to the main claim.

The present invention relates in particular to a panty or equivalent intimate garment of the type comprising an absorbent element or piece, to be positioned to correspond with the intimate parts of a woman (or "user") and adapted to absorb the fluid leakages from those parts. In particular, the absorbent piece is able to absorb leakages of small and medium extent.

Women of age between 40 and 45 years can suffer small episodes of urinary incontinence; these therefore require the use of an absorbent having the capacity to absorb these leakages more consistently than a common panty liner, but which at the same time is more practical than a usual absorbent to be used during the menstrual period, and is less bulky and more discreet than an article for senile incontinence.

Garments of the aforesaid type are already known. For example US6987210 relates to an intimate garment or panty comprising a single body formed of impermeable material and provided with an absorbent piece positioned to correspond with a portion of said body which lies in proximity to the intimate parts of the user.

The patent does not describe any characteristic of the absorbent piece and describes only the garment as formed of impermeable material. This garment is not completely comfortable for the user, particularly during warm periods of the year, or in crowded environments, or in all those situations in which the impermeability of this body, obtained by using silicone or latex which coats or constitutes the garment body itself except in that zone in which the absorbent piece is present, interferes with the normal free transpiration of the user's body.

US4355425 describes a panty having the features of the preamble of claim 1. However, this prior patent describes a panty body made of elastic rubber material; hence, this panty has the same drawbacks (relating to the uncomfortable use) of the intimate garment described in the above cited patent US6987210.

Moreover, US4355425 describes an absorbent or crotch element which has a skin-contacting porous thermoplastic topsheet, which therefore is of uncomfortable use for the user and which can give rise to skin irritation. This known panty cannot be washed.

US2005/022291 describes a protective undergarment which has the shape of a diaper and is used like a diaper and which cannot be used as a panty.

An object of the present invention is to provide an intimate garment provided with an absorbent piece which is able to absorb physiological liquid leakages at least of light-medium extent and is completely washable. Another object is to provide a garment of the stated type which is simple to produce and is completely similar to those intimate garments and the like (panty) without an absorbent piece, in terms of shape, of material, and of comfort in use.

Another object is to provide a garment of the stated type in which the absorbent piece presents a small thickness, such as not to display its presence.

These and other objects which will be apparent to the expert of the art are attained by an intimate garment or panty in accordance with the accompanying claims.

The present invention will be more apparent from the accompanying drawings, which are provided by way of non-limiting example and in which:
Figure 1 is a front view of a panty according to the invention;
Figure 2 is a partly exploded perspective view of a part of the product of

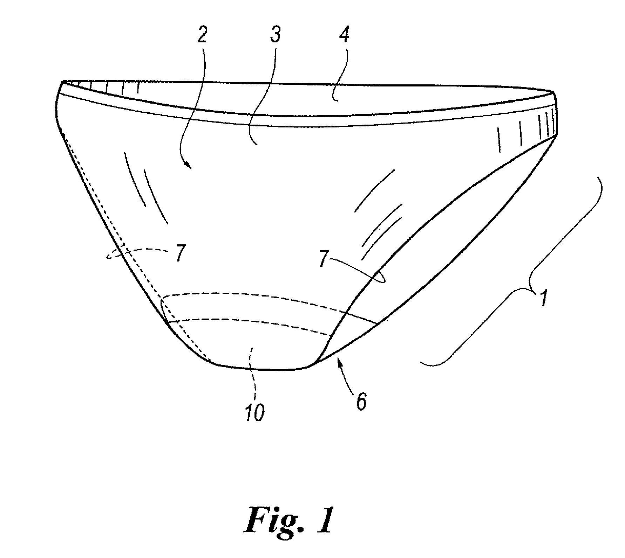

Figure 1; and

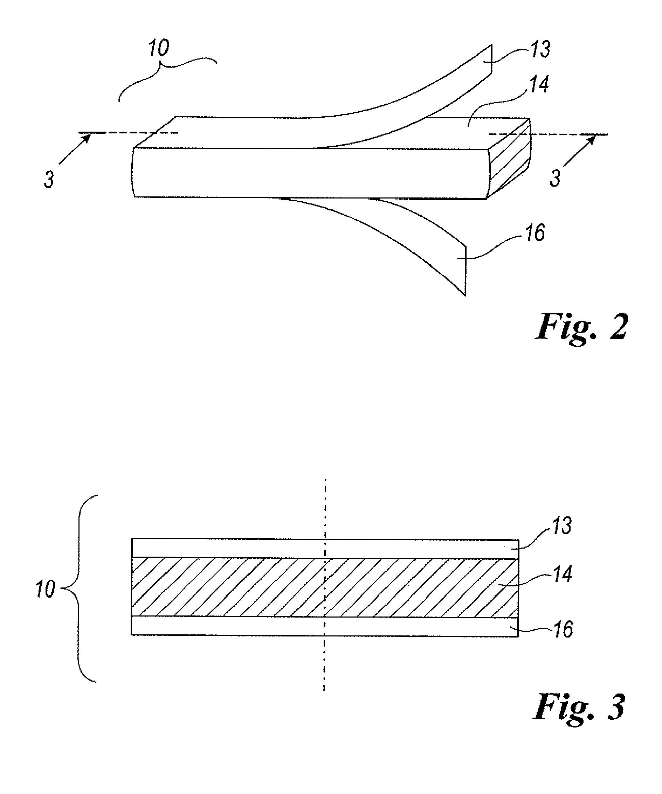

Figure 3 is a section on the line 3-3 of Figure 2.

With reference to said figures, the intimate garment or panty is indicated overall by 1 and comprises a waist portion 2 to be positioned about the waist of the user wearing it. This portion 2 comprises a front part 3 and a rear part 4 (with reference to their position when the garment is worn); an intermediate portion 6 of the garment 1 extends between the parts 3 and 4 and connects them together by passing over the user's crotch. Openings 7 are provided on the sides of the portion 6 for the user's legs.

In a position corresponding with the user's intimate parts, the intermediate portion 6 supports an absorbent piece 10 able to absorb any fluid leakages (for example urine) by the user. This piece is multi-layered but has a small thickness between 0.5 and 7 mm, advantageously between 2 and 5 mm and preferably between 3 and 4 mm, but such as not to be evident from outside the panty and not to annoy the user.

More particularly, the absorbent piece comprises a first layer 13 to enter into contact with the user's skin. This layer is of fabric, preferably of natural fibre (such as cotton, viscose or silk, for example). The layer 13 can advantageously be treated with an antibacterial and/or dermoprotective product to inhibit bacterial proliferation on contact with the skin, so preventing irritation and infection; the layer 13 has a high percentage absorption capacity (between 300 and 500, preferably between 465 and 489) and a surface density between 100 and 150 g/m², preferably between 110 and 120 g/m².

At least one second layer 14 forms the intermediate or internal layer of the absorbent piece 10. This layer is at least partly of wadding, to be understood in the widest sense of the term; wadding is a filamentous mass of fibres of average length between 5 and 55 mm. These fibres can be of cellulose wadding, can derive from cotton, can be of rayon or viscose or can be of non-woven wadding (known as silk-like wadding) formed of microfibre, polyester or a mixture of natural and/or synthetic fibres.

This layer has a high absorption capacity because of the high hygroscopicity of the wadding. This latter has a density between 100 and 500 g/m², preferably between 110 and 300 g/m², with a percentage absorption capacity between 1000 and 4800, advantageously between 2800 and 4000.

An outer third layer 16 of the piece 10 is of transpirable impermeable material such as polyester foil or the like. This layer has a density between 35 and 70 g/m².

The piece 10 is obtained by coupling together these layers. They are coupled together in known manner, for example by lamination with polyurethane foil, by thermowelding or the like, to form a single compact piece which is then connected to the intimate garment (of natural material such as cotton or the like) by sewing, thermowelding or insertion of a suitable pocket or seat provided to correspond with or provided in the intermediate portion 6 of the garment 1. This piece is preferably isolated from the panty by an external layer or film of impermeable polyester (or similar material), to prevent any marking of the user's clothes.

The garment 1, including the piece 10, can be washed by hand or by machine a large number of times (for example thirty at 40°C) without the piece 10 losing its absorption characteristics (layer 14), antibacterial characteristics (layer 13) and impermeability characteristics (layer 16). In particular, it has been shown that after dozens of washes at 50°-60°C the absorbent layer 14 maintained its absorption capacity substantially unaltered. A small percentage reduction in this absorption was found after the initial washes due mainly to the natural consolidation of the wadding fibres; however no further reduction was found after the subsequent washes.

A specific embodiment of the invention has been described. Others are however possible on the basis of the preceding description and are to be considered as falling within the scope of the ensuing claims.

## Claims

1. An intimate garment or panty comprising a body (1) with a waist portion (2) to be positioned about the waist of the user wearing it, said portion having a front part (3), a rear part (4), and an intermediate portion (6) which extends between the front part (3) and rear part (4) by passing over the user's crotch, opposing openings (7) being present on its sides for the user's legs, an absorbent piece (10) being positioned to correspond with the user's intimate parts to absorb any fluid leakages from said parts, the absorbent piece (10) comprising a plurality of superimposed layer elements (13, 14, 16), **characterised in that** the element for absorbing the fluid leakages is at least partly of wadding, the latter being a filamentous mass of fibres of average length between 5 and 55 mm, said body (1) being of natural material, the garmet or panty being completely washable.

2. An intimate garment as claimed in claim 1, **characterised in that** the layered piece (10) comprises a first outer layer (13) to make contact with the user's body, at least one intermediate layer (14) at least partly of wadding, and a second outer layer (16) of impermeable material, the piece (10) being fixed to the garment.

3. A garment as claimed in claim 2, **characterised in that** the first outer layer (13) is of fabric and presents a percentage absorption capacity between 300 and 500, preferably between 465 and 480, and a surface density between 100 and 150 g/m², preferably between 110 and 120 g/m²m, said fabric being either of natural fibre or microfibre.

4. A garment as claimed in claim 2, **characterised in that** the intermediate layer (14) can be of wadding chosen from cellulose wadding, cotton wadding, rayon or viscose wadding, or can be of non-woven wadding formed of microfibre, polyester or a mixture of natural and/or synthetic fibres.

5. A garment as claimed in claim 4, **characterised in that** the density of the intermediate layer is between 100 and 150 g/m², preferably between 110 and 120 g/m², and presents a percentage absorption capacity between 1000 and 4800, advantageously between 2800 and 4000.

6. A garment as claimed in claim 2, **characterised in that** the second outer layer (16) is of impermeable and transpirable material with a preferred density between 35 and 70 g/m².

7. A garment as claimed in claim 2, **characterised in that** the three layers (13, 14, 16) are fixed together, advantageously by lamination with polyurethane foil, or by thermowelding.

8. A garment as claimed in claim 1, **characterised in that** the absorbent piece has a thickness between 0.5 and 7 mm, preferably between 3 and 4 mm and advantageously between 2 and 5 mm.

9. A garment as claimed in claim 2, **characterised in that** the outer layer (13) is treated with an antibacterial product.

10. An absorbent piece to absorb any fluid leakage by a user and suitable to be positioned in correspondence with the user's intimate parts, in particular in a garment according to claim 1, said piece (10) being multi-layered and comprising a plurality of superimposed layers (13, 14, 16), one of this layers comprising an element suitable to absorb the fluid leakage, **characterised in that** said element is a least partly of wadding, the latter being a filamentous mass of fibres of average length between 5 and 55 mm, the adsorbent piece (10) being completely washable.

11. An absorbent piece as claimed in claim 10, **characterised in that** it comprises a first outer layer (13) to make contact with the user's body, this layer being fabric.

12. An absorbent piece as claimed in claim 11, **characterised in that** the first outer layer (13) presents a percentage absorption capacity between 300 and 500, preferably between 465 and 480, and a surface density between 100 and 150 g/m², preferably between 110 and 120 g/m²m, said fabric being either of natural fibre or microfiber.

13. An absorbent piece as claimed in claim 10, **characterised in that** the element suitable to absorb the fluid leakage is of wadding chosen from cellulose wadding, cotton wadding, rayon of viscose wadding, or non-woven wadding formed of microfiber, polyester of a mixture of natural and/or synthetic fibres.

14. An absorbent piece as claimed in claim 10, **characterised in that** the density of the element suitable to absorb the fluid leakage is between 100 and 150 g/m², preferably between 110 and 120 g/m², and presents a percentage absorption capacity between 1000 and 4800, advantageously between 2800 and 4000.
